# EUROPÄISCHE PATENTANMELDUNG

(11) **EP 3 266 471 A2**
(43) Veröffentlichungstag der Anmeldung: **10.01.2018**
(21) Anmeldenummer: 17000377.6
(22) Anmeldetag: 09.03.2017
(51) Int. Cl.: A61L 2/18

(54) **REINIGUNGSADAPTER UND SPÜLHÜLSE**

(30) Priorität: 12.04.2016 DE 102016004447
(71) Anmelder: Simmoteit, Robert, 72414 Rangendingen (DE)
(72) Erfinder: Simmoteit, Robert, 72414 Rangendingen (DE); Simmoteit, Kim-Louis, 69221 Dossenheim (DE)

(57) **Zusammenfassung**

Reinigungsadapter zum Reinigen von Produkten, insbesondere für die Produktaufbe-reitung von medizinischen Instrumenten, der über eine Zuleitung oder über einen Flüssigkeitsverteiler mit Reinigungsmedium versorgt wird und einen vorderen Spülraum (11) aufweist, um hiermit zu reinigende Produkte (3) oder Produktteile aufzunehmen, dadurch gekennzeichnet, dass zumindest ein Strömungshindernis im Adapter in Form von wand- und/oder bodenständige Materialausformungen (18, 38, 44) und/oder in Form eines räumliches Strömungshindernis (12, 19, 26, 29, 42, 45) vorhanden ist, um den Reinigungsflüssigkeitsaustritt aus dem Adapterkanal (14) so zu ändern, dass diese für die Produktkanal- und die Produktaußenreinigung eines Hohlraumproduktes oder zumindest für die Produktaußenreinigung mehrerer Produkte nutzbar ist. Dabei kann der Reinigungsadapter auch nur eine Hülse mit einem Anschlussteil und innenliegenden Teilen oder Ausformungen sein.

## Beschreibung

### Gebiet der Erfindung

Reinigungsadapter und Spülhülse zum Reinigen von Produkten, insbesondere für die Produktaufbereitung von medizinischen Instrumenten. Einsetzbar auch für Produkte, die im Labor- und in der Industrie gereinigt werden.

### Hintergrund der Erfindung

Die Reinigung und der Anschluss von medizinischen Hohlraumprodukten in Reinigungs- und Desinfektionsmaschinen werden immer wichtiger. Um Hohlraumprodukte sicher zu reinigen werden beispielsweise Reinigungsadapter und Spülhülsen in Reinigungsmaschinen eingesetzt. In diesen Spülhülsen oder Reinigungsadaptern werden die Produkte vorzugsweise senkrecht eingebracht und die von unten her kommenden Reinigungsmedien in die Produktkanäle eingeleitet. Die bekannten Spülhülsen bestehen in der Regel aus Kunststoff und sind einschraubbar. Eine Spülhülse zur Verteilung von Flüssigkeiten mit einer inneren Struktur ist in DE10 2012 022 261 beschrieben und besitzt einen innenliegenden Filter. In DE 10 2004 029 970 B3 und in DE 10 2008 050 991.4 werden Reinigungsadapter mit Spülverteiler kombiniert um Hohlraumprodukte zu reinigen. Reinigungsadapter können aber auch direkt über eine Zuleitung mit Reinigungsmedium versorgt werden.

Probleme bei der Hohlraumprodukte Reinigung ergeben sich immer wieder dadurch, dass die Produktreinigung nicht innen und außen gleichermaßen gut ist. Entweder werden die Produktkanäle gut gereinigt, da diese über einen Luer Lock-Anschluss optimal angeschlossen sind oder es erfolgt eine gute Außenreinigung der Produkte. Desweiteren wird die Reinigung von Produktkanälen in Hülsen durch deren Durchmesser bestimmt.

### Zusammenfassung der Erfindung

Die Erfindung hat die Aufgabe die bereits bekannten Spülhülsen und Reinigungsadapter zu verbessern. Es ist vorgesehen, medizinische Produkte sicher anzuschließen und/oder halten zu können und dabei zumindest Öffnungen von Hohlraumprodukte über einen Austrittskanal für Reinigungsflüssigkeit im Adapter sicherer anzuordnen und zu reinigen. Darüber hinaus ist eine Reinigungsvorrichtung vorgesehen, die die Reinigungsleistung durch einen neuartigen inneren Aufbau verbessert. Dabei soll der Reinigungsadapter oder die Spülhülse mit Vorrichtungen in Reinigungsmaschinen und/oder mit Siebkörben in Verbindung mit Spülverteilern kombinierbar sein. Das bevorzugte Einsatzgebiet der Neuerung ist die Aufbereitung von medizinischen Instrumenten mit mindestens einem Kanal sowie von schwierig zu reinigen Instrumentenspitzen.

Diese Aufgabe wird erfindungsgemäß durch einen Reinigungsadapter insbesondere für die Produktaufbereitung von medizinischen Instrumenten nach Anspruch 1 und durch eine Spülhülse nach Anspruch 13 gelöst. Folgende Ansprüche betreffen die vorteilhaften Ausgestaltungen der Erfindung.

Die Neuerung löst die Aufgabe durch einen Reinigungsadapter zum Reinigen von Produkten, insbesondere für die Produktaufbereitung von medizinischen Instrumenten, der über eine Zuleitung oder über einen Flüssigkeitsverteiler mit Reinigungsmedium versorgt wird und einen vorderen Spülraum aufweist, um hiermit zu reinigende Produkte oder Produktteile aufzunehmen, dadurch gekennzeichnet, dass zumindest ein Strömungshindernis im Adapter in Form von wand- und/oder bodenständige Materialausformungen und/oder in Form eines räumliches Strömungshindernis vorhanden ist, um den Reinigungsflüssigkeitsaustritt aus bzw. über den Adapterkanal so zu ändern, dass diese für die Produktkanal- und die Produktaußenreinigung eines Hohlraumproduktes oder zumindest für die Produktaußenreinigung mehrerer Produkte nutzbar ist.

Die Neuerung löst die Aufgabe insbesondere auch dadurch, dass dieser im inneren des Adapters ein Strömungshindernis zumindest in Ausgestaltung von Materialausformungen oder Einsätze mit Austrittsöffnungen oder -flächen (z. B. Gitter oder Lochscheiben) vorhanden sind, um zumindest die Flüssigkeitsverteilung im Adapter so zu ändern, dass ein Teil der Reinigungsflüssigkeit durch einen Produktkanal- und eine anderer Teil für die Produktaußenflächen nutzbar ist und/oder dabei ein Verschluss des Adapteraustrittskanals durch eines oder mehrere eingebrachten Produkte verhindert wird.

Die Neuerung sogt so dafür, dass nicht nur Hohlraumprodukte effektiv an den Außen- und Innenkanalflächen gereinigt werden, sondern ermöglicht auch, dass zumindest die Außenflächen mehrere Produktschäfte oder mehrerer Produkte erfolgen kann, ohne dass dabei ein Produkt den Adapterkanal verschließt und damit eine Reinigung zumindest der Außenflächen verhindert. Hierfür kann die Vorrichtung auch größer wie übliche als ein Hülsenadapter ausgeführt (Spülzylinderartig) sein, um z. B. im Spülraum mehrere Produkte gleichzeitig aufzunehmen und vorzugweise z. B. über einen zusätzlichen Halteeinsatz (z. B. eine Drahtschnecke oder Lochscheibe mit Halteöffnungen ect.) im Spülraum oder einen obigen Hülsenabschluss diese voneinander zu beabstanden und/oder zu halten. Von Vorteil ist, wenn sich die Außenflächen sich dabei nicht berühren. Das wiederum erhöht die Wirtschaftlichkeit der Instrumentenreinigung erheblich, da bisher nur ein Spülprodukt in einem hülsenförmigen Adapter sicher einsetzbar ist.

Die Neuerung besteht dabei aus einen Adapter der im inneren eine Zusatzeinrichtung/Strömungshindernis zur Strömungsänderung enthält oder der Adapter zumindest zweiteilig ausgeführt ist, damit eine Zusatzeinrichtung/Strömungshindernis leichter eingebracht und ausgetauscht werden kann. Im Letzteren Fall wäre das eine Scheibe mit geeigneten Materialaufformungen und Öffnungen, ein Gitter, eine Feder, eine Schnecke oder auch ein sich bewegendes Zusatzteil unter einer Scheibe oder einem Gitter zur Flüssigkeitsverwirbelung oder Umlenkung. Die bevorzugten Strömungshindernisse setzen dem einströmenden Reinigungsstrom einen Strömungswiderstand entgegen, bewirken dabei eine gewollte Beeinflussung der Strömung bis hin zur Erzeugung einer turbulenten Strömung im Adapterraum.

Von besonderem Vorteil dabei ist, wenn die in den Spülraum eintretende Reinigungsflüssigkeit zusätzlich verwirbelt wird, was die Reinigungsleistung signifikant verbessert. Die Verwirbelung sollte dabei auch noch bei relativ niedrigen Spüldrücke (Spüldrücke von 0,1 Bar- 1 Bar) funktionieren. Denkbar ist, dass das räumliche Strömungshindernis auch eine Ventilfunktion ausübt, um zumindest die austretende Reinigungsmenge in einem nicht genutzten Reinigungsadaptern zu reduzieren oder ganz zu unterbinden. Dabei kann der eingebrachte Instrumententeil die unteren Ventilklappen am Adapterboden herausstellen bzw. öffnen und einen Flüssigkeitseinstrom ermöglichen.

Als Strömungshindernisse werden dabei alle Strukturen definiert, die Flüssigkeiten lenken oder deren Bewegungsrichtung beeinflussen und/oder auch aufteilen können. Die neuartigen Reinigungsadapter besitzen innenliegende Strömungshindernisse, die eine Erhöhung oder eine Vertiefung einer Adapterwandung oder einen Einsatz mit Ausformungen und/oder Durchbrüchen beinhalten können, die die Bewegungsrichtung von Flüssigkeiten lenken, ändern und/oder aufteilen.

Dabei wird vorzugsweise ein trichterförmiges oder ein flächiges Strömungshindernis in Gestaltung von Materialvertiefungen oder Materialerhebungen und/oder z. B. ein Einsatz, der eine Spirale, eine Schnecke, ein Kegel, eine Feder, ein Gitter oder eine Scheibe sein kann, eingesetzt. Das innenliegende Strömungshindernis kann dabei auch eine Produkthalterung unterstützen, herstellen oder eine Produkthalterung sein oder eine Ventilfunktion ausüben. Dabei ist voreilhaft, wenn flächige oder kegelförmige Einsätze Durchbrüche oder Ausformungen besitzen, um den Flüssigkeitsstrom zu beeinflussen bzw. zu lenken und/oder zu verteilen.

Alternativ kann aber auch eine Kombination aus wandständigen Materialvertiefungen wie z. B. Fräsrillen (z. B. als Längsrillen) und Einsätzen Anwendung finden, um die Flüssigkeitsströme zumindest so aufzuteilen oder zu lenken, dass eine Außen- und Innenlumenreinigung und keine vollständige Überdeckung des Adapterkanals erfolgt. Diese Bedingungen sind Vorrausetzung für eine sichere und gute Reinigung von Hohlraumprodukten, insbesondere eine mittige und seitliche Flüssigkeitsverteilung.

Das Strömungshindernis kann dabei eine wellenförmige Ausformung und/oder eine Adapterwand mit Erhebungen oder Vertiefungen in Form von Fräsrillen besitzen. Hier können aber auch Abstandsnoppen oder Schrägrillen als wandständiges Strömungshindernis enthalten sein oder das Hindernis durch ein zusätzliches Teil, das in dem Adapter eingebracht wird, hergestellt werden. Alternativ ist aber auch denkbar, dass dieses Teil eine einschraubbare oder eine einschiebbare Hülse oder eine Spirale, eine Feder oder ein Formteil ist, welches sich nach unten hin trichterförmig verengt und dabei innere Erhebungen, Ausformungen oder seitliche Durchbrüche bzw. Kanäle aufweist. Denkbar ist aber auch, dass der Adapterboden flach ausgeführt ist und eine Spirale enthält, die nach oben verengt und nach unten verbreitert ist. Alternativ kann eine flache Scheibe mit Öffnungen mit Abstand über den Adapterkanal als Strömungshindernis eingebracht sein, um den Reinigungsstrom besser zu verteilen. Trichterförmig zulaufende Adapterböden, haben dabei den Vorteil, dass hier die eingebrachten Produkte besser über den Adapterkanal zentriert werden. Alternativ ist denkbar, dass das Strömungshindernis zumindest im Anschlusskanal des Adapters oder in einer Hülse ein bewegbares oder drehbares Teil in Form einer Kugel, eine Welle oder eine Turbine ist, um eine turbulente Strömung zu erzeugen. Beispielsweise könnte eine Kugel den zentralen Adapterkanal abdecken und nach oben durch den Reinigungsstrahl angehoben werden.

Die Erhebungen, Ausformungen oder Durchbrüche einer Hülse in einem Adapterraum verhindern, dass ein eingebrachtes Produkt den mittigen Kanalaustrittskanal vollständig überdeckt. Dadurch kann seitlich ausreichend Reinigungsflüssigkeit vorbeiströmen und für die Außenreinigung genutzt werden. Die Trichterform begünstigt dabei die mittige Anordnung des zu reinigenden Produktes. Die Neuerung sorgt somit dafür, dass eingebrachte Produkte oder Produktteile nach der Reinigung nicht mehr zusätzlich zumindest im vorderen Einsteckbereich nachgereinigt werden müssen. Ferner verbessert es die Reinigungssicherheit, so dass immer sichergestellt wird, dass eingebrachte Produkte eine verhältnismäßig gleichgute Innen- und Außenreinigung erfahren.

In Weiterbildung der Neuerung wird durch das Strömungshindernis in Ausgestaltung einer Spirale, Feder oder ähnliches und/oder einer Einschubhülse mit einer untenliegenden Verengung vorzugsweise eine Turbulenz in der austretenden Reinigungsflüssigkeit erzeugt. Durch die angeregte zusätzliche Flüssigkeitsbewegung werden innenliegende und außenliegende Instrumentenoberflächen effizient gereinigt. Die Reinigungsleistung hängt dabei von der Einströmungsgeschwindigkeit der Reinigungsflüssigkeit und die durch das Hindernis erzeugt Turbulenz ab.

Die Neuerung stellt dabei sicher, dass in dem Reinigungsadapter und/oder in einer Spülhülse die hier eingebrachten Produktteile die Austrittsöffnung des Adapters nicht dichtend überdecken und durch eine geänderte Flüssigkeitsverteilung die Reinigungseffizient von Hohlraumprodukten verbessert wird. Das wäre jedoch bei dem bekannten Stand der Technik nicht der Fall, da hier durch die Gewichtskraft eines zu reinigenden Instruments eine mittig angeordnete Adapteröffnung ganz überdeckt und wenig oder gar keine weitere Flüssigkeit mehr zur Außenreinigung zur Verfügung steht. Auch wenn zusätzlich zum mittig angeordneten Adapterkanal noch seitliche Austrittsöffnungen vorgesehen wären und diese nicht in einem Trichter ohne Abstandshalter zum Adapterkanal angeordnet sind, wäre damit nicht völlig sichergestellt, dass eine sinnvolle Aufteilung der Reinigungsflüssigkeit erfolgt. Es gilt dabei zu beachten, dass die Innenlumen-Reinigung Vorrang vor der Außenreinigung hat und das insbesondere dann, wenn nur eine begrenzte Reinigungsflüssigkeitsmenge zur Verfügung steht. Hier muss man sich vor Augen führen, dass der Durchmesser der Austrittsöffnungen vom Reinigungsadapter in der Regel 2 - 4 mm beträgt, hingegen die zu reinigenden Instrumentenkanäle sehr oft deutlich größer als diese Austrittsöffnungen sind. Eine Größenreduktion der technisch gut nutzbaren Adapterkanäle durch ein Innenteil hat den Vorteil, dass hier kein dünnes Instrumententeil hineinrutscht. Ferner ist es sinnvoll, eher kleine Zuführkanäle einzusetzen, da in Reinigungsmaschinen nur eine begrenzte Reinigungsflüssigkeitsmenge zur Verfügung steht. Wichtig ist aber auch für Flüssigkeitsverteiler (z. B. Spülleisten oder Spülzylinder), dass der benötigte Reinigungsdruck erhalten bleibt.

In Weiterbildung sieht die Neuerung einen einschraubbaren Reinigungsadapter auch für liegende Produkte in der Art vor, dass ein verlängerter Spülraum vorzugsweise vorne in der Vorrichtung eine gezackte Öffnung besitzt, die auch über eine untere Klappe verschlossen werden kann. Im letzteren Fall ist eine zusätzliche kleine Entlastungsöffnung vorgesehen, die verhindert, dass Reinigungslösung im vorderen Raum während der Reinigung verbleibt und nicht ausgetauscht wird. Spülhülsen können als Ergänzungsteile vorzugsweise mit einem Metalladapter lösbar verbunden werden und nach unten hin zusätzlich ein Flüssigkeitsfilter enthalten, um unerwünschte Partikel zurückzuhalten.

Es ist denkbar, dass die Spülhülse selbst den Reinigungsadapter bildet, ein Anschlussteil besitzt und die Reinigungsleistung von medizinischen Hohlraumprodukten und/oder für mehrere Produktflächen die sich in einer Spülhülse befinden können, verbessert. Der Anschlussteil kann ein Gewindeansatz, ein Luer Lock, ein Schlauchanschluss oder ein Steckteil sein, um hierüber Reinigungsflüssigkeit in den Adapter bzw. die Hülse zu leiten. Nach vorne ist die Hülse so lang ausgebildet, dass sie eingebrachte Produkte sicher hält und damit auch eine Halterungshülse sein kann.

Die Neuerung löst die Aufgabe auch durch eine Spülhülse in Ausgestaltung eines Reinigungsadapters, zum Reinigen von Produkten insbesondere für die Produktaufbereitung von medizinischen Instrumenten, die ein Anschlussteil oder eine Zuleitung für Reinigungsflüssigkeit (z. B. Schlauch) aufweist und zu reinigende Produkte oder Produktteile aufnimmt, dadurch gekennzeichnet, dass die Spülhülse Erhebungen am Spülhülsenboden oder überströmbare innenliegende Teile oder ein herausstehendes Rohrteil mit seitlichen Öffnungen besitzt um Reinigungsflüssigkeit zu verteilen oder zumindest zu verwirbeln, um damit die Produktkanal- und Produktaußenreinigung zu verbessern.

Die Spülhülse kann auch dadurch gekennzeichnet sein, dass die aufgenommenen Produkte durch eine innenliegende Produkthalterung (z. B. eine Haltefeder) gehalten werden und gleichzeitig diese Halterung die Reinigungsflüssigkeit aus dem mittigen Anschlusskanal verteilt und verhindert, dass diese den Anschlusskanal überdeckt. Dabei kann eine innenliegende und elastische Hülse, welche auf die Feder seitlich drückt, die Haltekraft zusätzlich erhöhen oder verbessern.

Als eine andere Weiterbildung kann die eingesetzte Spülhülse selbst eine Halterungsfunktion ausüben. In Verbindung mit einem Reinigungsadapter oder als eigenständige Reinigungshülse kann sich der Hülsenmantel des Adapters, zumindest nach innen, wie eine Düse verengen. Diese Ausgestaltung erleichtert das Einführen von zu reinigenden Produktteilen und stellt gleichzeitig eine Stützstruktur für lange Instrumente bereit. Weitere Vorteile ergeben sich dadurch, dass dabei das Produkt über den Hülsenschaft sich zusätzlich mittig im Adapterraum anordnet. Besitzt die Hülse durchgängig Erhebungen z. B. in Form von Längs- oder Querrillen an der Innenwandung, so wird die Erzeugung von turbulenter Strömung im Inneren der Hülse verbessert.

Die Spülhülse kann aber auch ein einfaches Rohr mit einem Flüssigkeitsanschluss sein, das ein innenliegendes Gitter zur Filterung oder als Strömungshindernis besitzt oder hier wiederum eine zusätzliche Hülse sein, die wiederum in eine Spülhülse mit innenliegenden Strukturen eingebracht ist. Durch diese Hülsenanordnung (Hülsenkonzept) wird die Spülhülse vielseitig nutzbar und wirtschaftlich in seiner Herstellung.

Als eine Neuerung kann zumindest der einschraubbare oder verbindbare Adapterteil einen Adapterraum zur Aufnahme von Spülhülsen eine innenliegende Welle als Strömungshindernis aufweisen. Da deren Herstellung in Kanälen aufwendig ist, wird hier eine zumindest trichterförmige Spirale oder Feder oder ein Produkthalterung vorgeschlagen, die nachträglich in einem Adapterraum eingebracht wird. Metallspiralen aus Draht haben den Vorteil, dass diese verhindern, dass eingebrachte Produktschäfte keinen direkten Kontakt mit der Adapterwandung mehr haben können. Von Vorteil sind dabei flächig und leicht trichterförmige Spiralfedern. Alternativ kann der gesamte Reinigungsadapter aus einem Stück gefertigt sein.

Als geeignete Strömungshindernisse sind anstelle von Spiralen oder Federn auch Spritzgussteile denkbar, die die Beweglichkeit von hindurchtretenden Flüssigkeiten zur Produktreinigung positiv ändern. Weitere mögliche Hindernisstrukturen können insbesondere sternförmige Austrittsöffnungen oder drehbare Kugeln in Käfigen besitzen.

Als Weiterführung der Erfindung kann der neue Reinigungsadapter oder die Spülhülse eine so große vordere Öffnung und Größe aufweisen, dass hierin Dental- oder medizinische Motorhandstücke eingebracht werden können. Die Gestaltung der vorderen Öffnung z. B. der Hülse kann auf die zu reinigenden Produkte abgestimmt werden, um dadurch eine verbesserte Innenreinigung und Außenreinigung komplexer medizinischer Produkte zu ermöglichen. Alternativ können zumindest Teile der Neuerung auch ausschließlich zur Außenreinigung von Produkten in der Art eingesetzt werden, dass diese an Dreharmen angebracht sind und Reinigungsmedium flächig versprühen.

Damit die Neuerung wirtschaftlich einsetzbar ist, sind Reinigungsadapter zu bevorzugen, deren Aufbau mehrteilig, flexible und modular sind und diese an Maschinenleisten von Reinigungsmaschinen wie auch in Verbindung mit Flüssigkeitsverteilern wie Spülleisten der Spülzylinder, zum Reinigen von medizinischen Hohlraumprodukten einsetzbar sind. Dabei besitzt der Reinigungsadapter vorzugsweise einen vorderen änderbaren und als Anschluss für Reinigungslösungsleitungen ein Luer Lock, Gewinde-, Bajonett- oder Steckteil. Dieser Aufbau gewährleitet insbesondere durch den Austausch auch der innenliegenden Teile die Reinigung von kleinlumigen sowie auch von großlumigen Produkten, ohne dass das Adapterdesign an sich geändert werden muss. Der vordere änderbare Teil kann ein Kunststoffteil in Ausgestaltung einer längeren Hülse oder als ein Trichter ausgebildet sein. Der Querschnitt des Reinigungsadapters ist bevorzugt rund, kann aber auch rechteckig oder anders geformt sein.

### Einsatzgebiete und Ausführungsformen

Es ist vorgesehen den Reinigungsadapter so zu nutzen, dass das Spülen, Anschließen und/oder auch das Halten von Hohlraumprodukten und deren Reinigung verbessert wird. Ferner ist es möglich, je nach Gestaltung der Hülse, auch kleinlumige Produkte wie Augeninstrumente, HNO-Sauger, Saver-Blads, Dentalbohrer zu reinigen. Je nach Bauart des Adapters können auch Dentalhandstücke und großlumige Hohlraumprodukte innen wie auch außen effizient gereinigt werden. Das Einsatzgebiet ist jedoch nicht ausschließlich auf medizinische Produkte beschränkt.

Für folgende Anwendungsbereiche lässt sich der Reinigungsadapter oder die Spülhülse einsetzen:
1. Hohlrauminstrumente aller Art.
2. Produktreinigung von senkrecht stehenden Produktteilen und Produkten
3. Produktreinigung von liegenden vorzugsweise schräg stehenden Produkten oder Produktteilen durch zusätzlich innenliegende, herausstehende oder abschließende Halteeinsätze.
4. Produktreinigung von mehreren Produkten mit oder ohne einen Kanal, die im Adapterraum eingebracht sind.
5. Produktreinigung mit einer Verengung oder durch Ausformungen im Hülsenschaft zur besseren Halterung der zu reinigenden Produkte oder zur Erzeugung eines Venturi-Effekts.
6. Produktreinigung mit Filterung der Reinigungsflüssigkeit durch einen Filter oder ein Gitter welches unmittelbar vor einem Austrittskanal angeordnet ist.
7. Produktreinigung in Reinigungsmaschinen sowie in Ultraschallbädern.
8. Modulare Reinigungstechnik durch individuelles Einbringen von Reinigungsadapter und/oder Spülhülsen am Rohrverteiler oder ähnliches durch ein Schraubgewinde oder ein Luer Lock, Bajonett-, Schlauch- oder ein Steckteil.
9. Modulare Reinigungstechnik durch Einbringen von Einsätzen in Ausgestaltung von Scheiben, Zylindern, Trichtern, Spiralen, Federn oder drehbaren Teilen.
10. Schonende Produktbehandlung durch weiche Silikonhülsen oder innenliegende z. B. weiche Adapterteile, um zu reinigende Produktteile im Spitzenbereich nicht zu beschädigen.
11. Verbesserte und sichere Flüssigkeitsteilung durch innenliegende Ausfräsungen oder Ausformungen und damit verbunden keine Überdeckung des mittleren Adapterkanals.
12. Eine wirtschaftliche und verbessere Innen- und Außenlumenreinigung durch austauschbare Halterung für Spülhülsen.
13. Verbessere Produkthalterung durch Öffnungen, die beispielsweise elastische Halterungsstege aufweisen oder sternförmig ausgebildet sind und/oder durch eine innenliegende Haltefeder oder durch zusätzliche Ventilklappen ein Produkt fixieren.

In bevorzugter Ausgestaltung besitzt der Reinigungsadapter ein hinteres kurzes Gewinde zum Einschrauben in einem beweglichen Flüssigkeitsverteiler oder an einer Maschinenleiste. Nach vorne ist eine 2 - 20 cm lange Hülse vorgesehen. Die Hülse kann vorne verengt sein oder z. B. eine gezackte bzw. unförmige Öffnung aufweisen. Innenliegend ist eine vorzugsweise trichterförmige zulaufende Drahtspirale zur besseren Flüssigkeitsverteilung eingesetzt. Dabei kann die Drahtspirale nach unten ein verlängertes Drahtstück aufweisen, das direkt und vorzugsweise mittig in den Adapterkanal eingebracht ist. Anstelle des verlängerten Drahtstücks im Kanal kann hier auch ein kugliges Ende vorgesehen sein, um Flüssigkeit umzulenken oder zu verteilen. Bevorzugt sind trichterförmig ausgebildete Drahtspiralen, Gitter oder Formteile, die in trichterartige Adapteraustrittskanäle passen. Dieser Aufbau reduziert die Adapterkanalöffnung und ändert die Flüssigkeitsströmung je nach Ausführungsart.

Eine weitere technische Ausgestaltung des Adapters ist, dass von außen eine Hülse in ein bereits vorhandenes hülsenförmiges Adapterteil eingeschoben wird. Die Hülse kann nach unten so ausgebildet sein, dass sich hier eine Verengung und/oder sich ein Drahtgitter befindet. In Weiterbildung kann die Hülse ein zusätzliches Halteteil, beispielsweise in Kombination einer außen oder innenliegend Klemmfeder aufweisen. Die Haltefeder wird entweder durch die eigene Rückstellkraft oder durch die Rückstellkraft einer elastischen Kunststoffhülse (z. B. einer Silikonhülse) zusammengedrückt. Dadurch wird ein in den Hülsenraum eingebrachtes Instrument über eine Anpresskraft gehalten.

Es ist weiter denkbar, dass Lochscheiben, Kegel mit mittig und seitlich angeordneten Austrittsöffnungen vorgesehen sind, die in Adapterhülsen oder Adapterräume eingebracht sind. Damit die Scheiben oder Kegel nicht durch den Flüssigkeitsdruck herausgedrückt werden, ist im Adapterraum eine Aufnahmenute vorgesehen.

In einer anderen Ausgestaltung werden räumlich angeordnete flächige Spiralen im Innenraum eines Adapters als Strömungshindernis eingesetzt. Diese wird über eine Innennute im Adapter vorzugsweise bodenständig und beabstandet vom Einströmkanal angeordnet. Alternativ kann diese Spirale auch direkt am oder sogar im Adaptereinströmkanal angeordnet sein und diese einengen.

Andere Ausgestaltungen des Adapters werden erreicht, wenn dieser vorne und/oder hinten Gewinde aufweist. Hieran können dann die unterschiedlichsten Adapterteile verschraubt werden. Diese Gestaltungsmöglichkeiten ändern die Funktion und den Verwendungszweck des Adapters wesentlich.

Eine bevorzugte Ausgestaltung ist eine Spülhülse die nach unten hin ein Steck- oder ein Schlauchteil zum Anschluss an einem Spülrohr oder an einem Spülschlauch aufweist. Die Hülse besitzt im Boden einen Trichter und seitliche Erhebungen in Form von Längsrillen. Diese können auch Noppen sein. Rillen oder Noppen verhindern z. B. einen vollständigen Verschluss des mittigen Austrittskanals der Spülhülse durch ein von oben eingeführtes Instrument. Werden derartige Hülse ganz aus weichem Silikon hergestellt, so lassen sich die Hülsenschäfte durch eine Manschette im Innenlumen verengen.

Weitere Vorteile der Erfindung ergeben sich aus der Wahl der kombinierten Bauteile und Materialien sowie aus der Beschreibung und den beigefügten Zeichnungen.

Es versteht sich, dass die vorstehenden genannten und die nachstehenden noch zu erläuternden Merkmale nicht nur in der jeweils angegebenen Kombination, sondern zum Teil auch in anderen Kombinationen oder in Alleinstellungen verwendbar sind. Ausführungsbeispiele der Erfindung sind in den Zeichnungen dargestellt und werden in den nachfolgenden Beschreibungen näher erläutert.

Es zeigen:
- Fig. 1.: Draufsicht möglicher Austrittsflächen für Spüllösung in den Adapterraum
- Fig. 2.: Reinigungsgewindeadapter und eine innenliegenden Spirale.
- Fig. 3.: Teildarstellungen von innenliegenden Strömungshindernissen.
- Fig. 4.: Draufsicht von Adapter-, Spülhülsen- oder Kappenöffnungen.
- Fig. 5.: Gestaltungsformen von einsetzbaren trichterförmigen Strömungshindernissen.
- Fig. 6.: Spülhülse mit Anschlussteil.

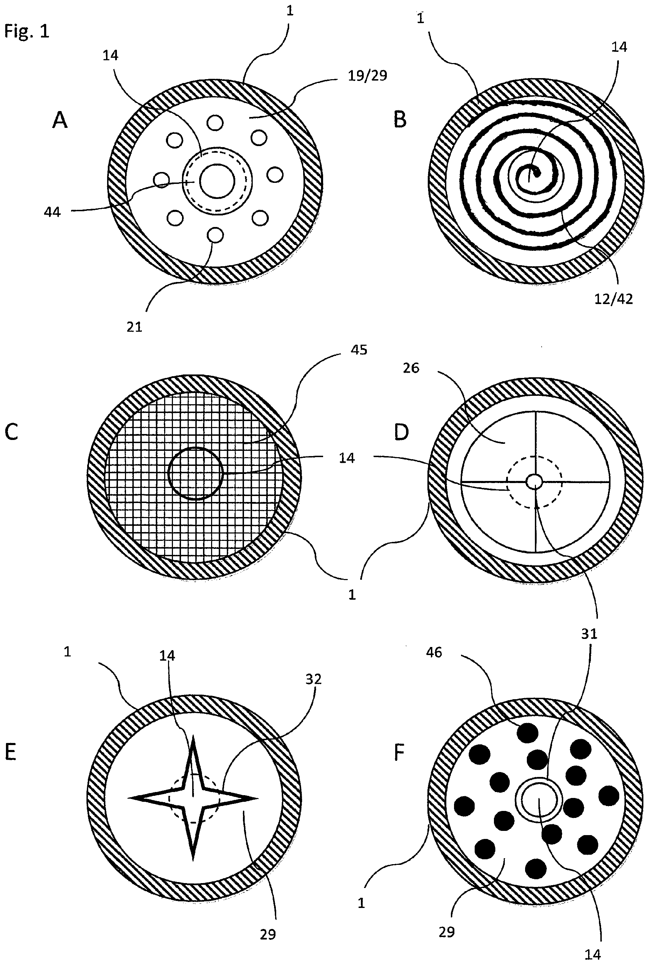

Figur 1. Zeigt in Draufsicht möglicher Austrittsflächen für Spüllösung in den Adapterraum. In **A** ist in Draufsicht eines Reinigungsadapters **1** gezeigt in eine Scheibe **29** als Einsatz **19** die ein Strömungshindernis bildet, welche vorzugsweise beabstandet einen Adapterkanal **14** überdeckt. In der Scheibe **29** sind kleine Öffnungen **21** und eine zentrale größere Öffnung **44** vorhanden. Dabei kann die größere zentrale Öffnung **44** vorzugsweise auch im Randbereich erhöht ausgebildet sein. Wird hier der Schaft eines Hohlrauminstrumentes eingebracht, so wird auch bei Schrägstellung des Schaftes der innere Hohlraum durchspült oder bei senkrechter Stellung je nach Größe auch die Außenflächen mit Reinigungsflüssigkeit versorgt. Die Abbildung **B** zeigt in Draufsicht einen Reinigungsadapter **1** mit einem Adapterkanal in dem eine Feder bzw. eine Drahtspirale **12** oder eine Feder **42** als Strömungshindernis **12** beabstandet über einen Adapterkanal **14** eingebracht ist. Der Drahtfortsatz ist dabei mittig in den Kanal **14** angeordnet. Wir über diese Spirale **12** (Strömungshindernis) Reinigungsmedium geführt so wird diese in eine Turbulenz versetzt. Dadurch wird ein Teil des Reinigungsmediums in Turbulenz einen Instrumentenkanal durchspülen können und ein anderer Teil des Reinigungsmediums an der Instrumentenaußenfläche geführt. Eine weitere Draufsicht **C** zeigt einen Adapter in dem im Innenraum ein Gitter über einen Adapterkanal **14** angeordnet ist. Hier ist das Gitter **45** das Strömungshindernis und sorgt dafür, dass ein Instrumentenschaft nicht den Adapterkanal **14** vollständig verschließen kann. Die Darstellung **D** zeigt in Draufsicht einen Reinigungsadapter, bei dem ein Ventileinsatz **26** mit Klappen als Strömungswiderstand den Adapterkanal **14** mit einem Abstand zum Adapterboden überdeckt. Um die Ventilklappen nicht zu stark herauszudrücken besitzt das Ventil **26** eine zentrale Öffnung **31**. Wird hier ein Instrumentenschaft eingebracht, öffnen sich die Ventilklappen nach unten hin. Die Abbildung **E** bildet in Draufsicht einen Reinigungsadapter ab, der eine Scheibe aufweist, die in der Mitte eine sternförmige Öffnung **32** besitzt. Die so ausgebildete Scheibe **29** ermöglicht ebenfalls, dass Reinigungsmedium nicht nur mittig über den Adapterkanal **14** ausströmen kann. Denkbar ist hier, dass eine unter der Scheibe **29** liegende Kugel durch den Reinigungsstrahl angehoben wird und dadurch eine turbulente Strömung in Verbindung mit der sternförmigen Öffnung **32** begünstigt. In der Darstellung **F** ist wiederum eine Scheibe **29** gezeigt, auf deren Fläche mittig eine Öffnung **31** und Abstandsnoppen **46** ausgebildet sind. Die Abstandsnoppen **46** sorgen dafür, dass Instrumententeile beabstandet vom Scheibenboden angeordnet werden. Alternativ, kann auch nur der Adapterboden diese oder andere Abstandshalter aufweisen, um die aus dem Adapterkanal ausströmende Reinigungslösung besser im Adapterraum zu verteilen.

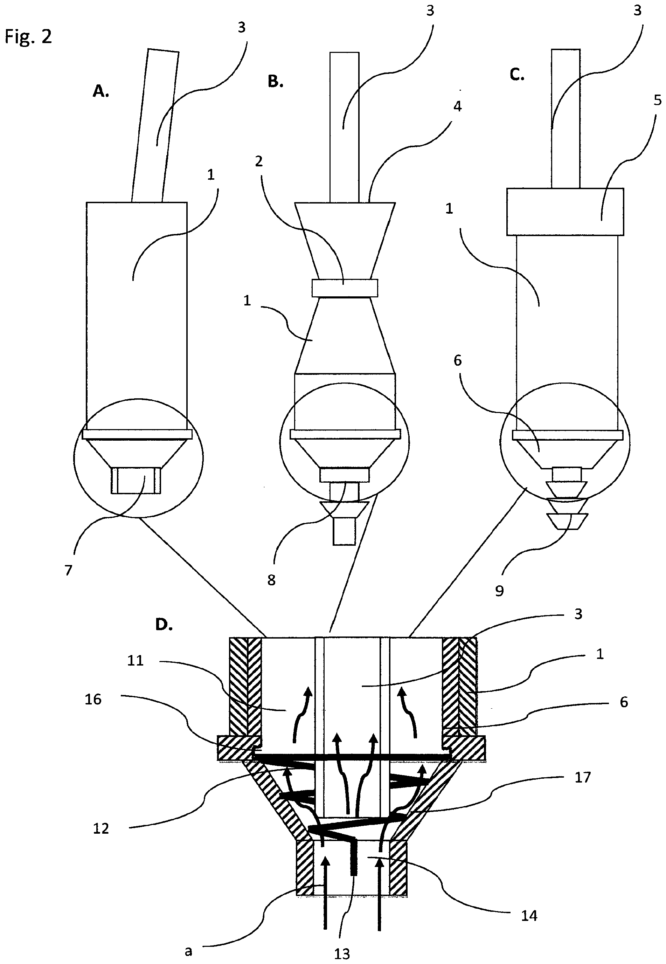

Figur 2. zeigt Reinigungsgewindeadapter **A, B,** und **C** mit einer innenliegenden Spirale. Die Abbildung **A** zeigt einen Reinigungsadapter der nach unten einen Gewindeansatz **7** zum Festlegen des Adapters an einem Spülverteiler und nach oben hin eine Hülse **1** aufweist, in der ein Instrument **3** eingebracht ist. Da die obere Hülsenöffnung groß ist, kann das Instrument hier auch schräg stehen. Die Abbildung **B** zeigt einen Reinigungsadapter, bei dem der Hülsenteil **1** durch eine Manschette **2** eingeengt ist. Die obere Adapteröffnung **4** ist dadurch reduziert und verbesserte das mittige Einführen eines Instruments **3**. Nach unten hin besitzt dieser Reinigungsadapter einen Steckanschluss **8**. Der Reinigungsadapter **C** besitzt einen Schlauchansatz **9**, um hierüber Reinigungslösung einzuleiten, ein Hülsenteil **1** und eine obere Kappe **5**, mit der ein Instrument **3** mittig im Adapter gehalten wird. Die Anschlussteile **7**, **8** und **9** der Reinigungsadapter können mit den Hülsen **1** lösbar verbunden sein. Die Darstellung **D** zeigt in Schnittdarstellung den unteren Adapterteil **6** der obigen Reinigungsadapter **A**, **B** und **C** mit einem innenliegenden Kanal **14**, der sich in dem Spülraum **11** trichterförmig öffnet und eine Hinterschneidung **16** zur Aufnahme eines Strömungshindernisses **12** besitzt. Das gezeigte Hohlrauminstrument **3** ist dabei mittig eingeführt und kann die innenliegende Spülraumwandung am Adapterboden **17** nicht flächig berühren und abdichten. Dies verhindert die innenliegende trichterförmige Spirale **12**, die nach unten hin eine Verlängerung **13** besitzt. Die von unten her einströmende Reinigungsflüssigkeit (dargestellt durch die Pfeile a) in dem Adapterkanal **14** wird durch das Strömungshindernis **12** umgelenkt und so verteilt, dass diese in den Innenkanal des Hohlrauminstrumentes und außen am Instrumentenschaft verbeiströmen kann. Die Verlängerung der Spirale **13** verbessert dabei die Strömungsumlenkung und kann je nach Ausgestaltung (kugliges oder kegliges Endstück) und in Abhängigkeit des Spüldrucks einen turbulenten Reinigungsstrom erzeugen. Es ist gut zu erkennen, wie das innenliegende Strömungshindernis bzw. die Spirale **12** an der zylindrischen Adapterseitenwand **17** (Adapterboden) anliegt und nicht nur den Flüssigkeitsstrom ändert, sondern auch effizient verhindert, dass das Hohlrauminstrument oder ein großer Instrumentenschaft den Adapterkanal **14** vollständig überdeckt, insofern dieser nicht größer als der Spülraumdurchmesser ist und der Spülraum z. B. durch seine Elastizität diesen aufnehmen kann. Der Adapterboden **17** kann dabei wie gezeigt trichterförmig aber auch flach ausgeführt sein. Nach unten hin ist der Adapterteil **6** mit einer Hülse **1** lösbar verbunden und bildet einen langen Spülraum **11**. Die Länge des Spülraums lässt sich so durch die Hülsenlänge ändern.

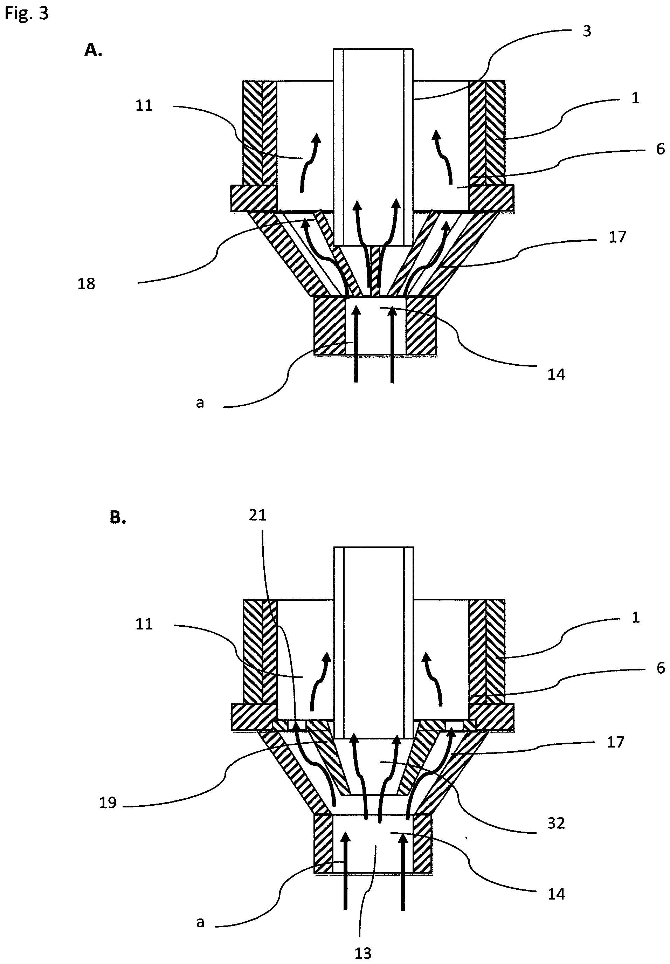

Figur 3. zeigt in **A** und **B** Teildarstellungen von innenliegenden Strömungshindernissen. In **A** befinden sich am Adapterboden **17** Vertiefungen **18** in Ausgestaltung von Längsrillen. Diese können durch Ausfräsungen hergestellt werden oder durch ein entsprechendes Hülsenteil eingebracht sein. Der untere Adapterteil **6** dient dabei zur Aufnahme einer Hülse **1**, die nach oben hin den Adapterinnenraum **11** vergrößert. Alternativ kann dieser Raum **11** auch nur der Reinigungsadapter selbst bereitstellen. Ein in dem Adapterraum **11** eingebrachtes Hohlrauminstrument **3** liegt so am Adapterboden **17** an, dass Reinigungsflüssigkeit (durch **Pfeile a** angedeutet) durch den Instrumentenkanal und seitlich am Instrumentenschaft strömen kann. Die Darstellung **B** zeigt eine andere Variante eines Strömungshindernisses in Ausgestaltung eines Trichtereinsatzes **19** mit seitlichen Durchbrüchen **21** und einen mittigen Kanal **32**. Auch dieser Kanal kann Ausfräsungen oder andere Erhebungen, wie in Abbildung **A** gezeigt, besitzen. Dieser Trichtereinsatz kann alternativ auch durch einen zweiteiligen Adapter bereitgestellt werden und zwar durch ein obiges verlängertes Teil mit einem Trichter **19** und nach unten hin einem Adapterteil **6** mit einem Anschluss für Flüssigkeiten. Auch hier ist deutlich zu sehen, wie ein eingebrachtes Hohlrauminstrument **3** in dem Innenraum **11** nicht den Adapterboden **17** berührt und den mittigen Adapterkanal **14** zwar überdeckt, jedoch durch den Einsatz **19** die äußere Flüssigkeitsverteilung (Pfeile a) seitlich ermöglicht und das Holrauminstrument **3** vollständig (die Innenkanalflächen und die Außenflächen) erreicht.

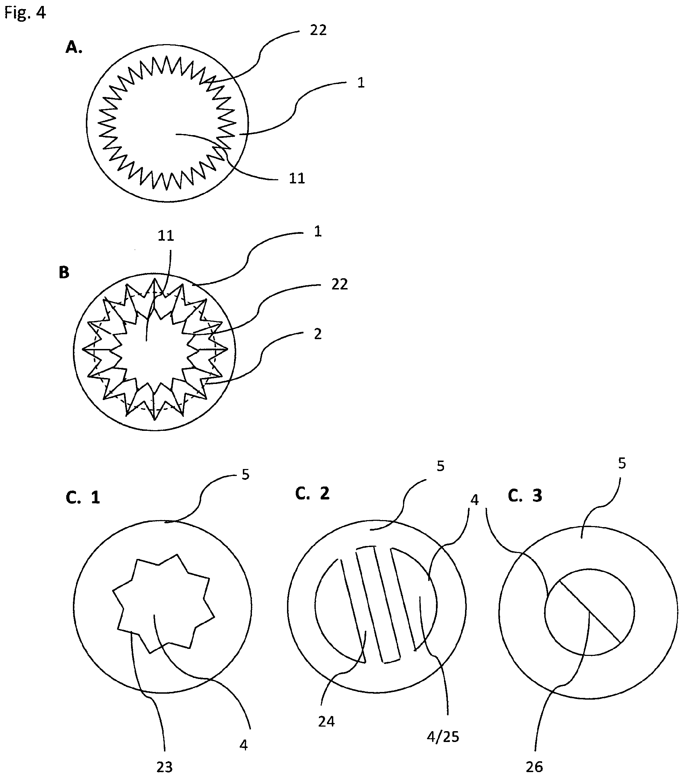

Figur 4 zeigt eine Draufsicht in **A, B** und **C** Adapter-, Spülhülsen- oder Kappenöffnungen. Die Darstellung **A** zeigt eine Hülse **1** mit einer zackigen Innenwandstruktur **22**. Diese kann alternativ auch noppenartig oder anders gestaltet sein. Hat jedoch zum Ziel, den Kontakt eines eingebrachten Instruments mit der Hülsenwand so zu gestalten, dass immer seitlich an der Hülsenwandung Reinigungsflüssigkeit strömen kann, um das Instrument im Hülsenraum **11** zu reinigen. Die Darstellung **B** zeigt eine weitere Hülse **1** mit innenliegenden Wandausformungen **22**. Der Hülsenraum **11** wird dabei über eine Manschette **2** eingeschnürt. Alternativ kann die Hülse auch in dieser Form ohne Manschette hergestellt sein. Diese Ausgestaltung verbessert das mittige Einführen und Halten eines Instruments und verhindert, dass zu große Instrumentendurchmesser in die Hülse eingeführt werden können. Die Abbildungen **C1** bis **C3** zeigen unterschiedliche Kappen **5** mit Öffnungen **4**. In **C1** ist die Öffnung **4** sternförmig oder unrund **23** ausgebildet ist. In **C2** befinden sich zwei Stege **24**, die parallel die Öffnung **4** überdecken. Dabei kann die Öffnung **4** der Kappe **5** eine untere Klappe **25** überdecken und so verhindern, dass hier Reinigungsflüssigkeit austritt, wenn hier kein Instrument eingeführt ist. Die gezeigte Kappe kann dabei aus einem elastischen Material wie z. B. Silikon bestehen. In dieser Form können sich die Stege **24** seitlich dehnen und so einen Instrumentenschaft halten. Ähnlich kann in **C3** eine andere Kappe mit einer Öffnung **4** ein Klappenventil **26** mit zwei Klappen überdecken oder selbst ein Ventil **26** sein und einen ungewollten Flüssigkeitsausstrom zumindest reduzieren, wenn kein Instrument eingebracht ist.

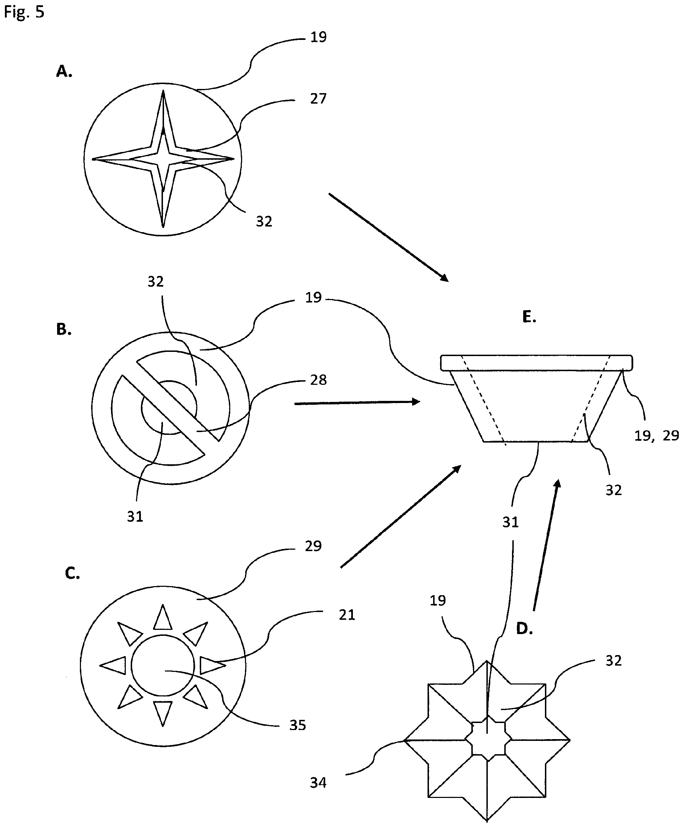

Figur 5. zeigt unterschiedliche Gestaltungsformen **A**, **B**, **C** und **D** von einsetzbaren trichterförmigen Strömungshindernissen. Die Darstellung **A** zeigt in Draufsicht ein Strömungshindernis **19** mit einer unteren Öffnung und einer oberen vergrößerten sternförmigen Öffnung **27**. Der so gestaltete stern- und trichterförmig Kanal **32** des Strömungshindernisses **19** verteilt die Reinigungsflüssigkeit mittig wie auch zur Seite und verbessert so die Reinigungsleistung in dem Spülraum eines Reinigungsadapters. In der Darstellung **B** ist ein anderes Strömungshindernis **19** in Ausgestaltung eines trichterförmiges Kanal **32** gezeigt, der mittig durch eine Wandung **28** geteilt ist. Alternativ können hier auch zwei oder mehrere Wandungen eingebracht sein, um den Flüssigkeitsstrom aufzuteilen. Die Darstellung **C** wiederum kann als eine Scheibe **29** mit einer mittigen Öffnung **35** und seitlich angeordneten Durchbrüchen **21** oder Kanälen ein Strömungshindernis bilden oder ein zusätzlicher Bestandteil eines unteren trichterförmiges Hindernisses (Spirale, Kegel, trichterförmige Hülse usw.) sein. Alternativ ist in **D** ein weiteres trichterförmiges Strömungshindernis **19** denkbar, das sowohl innenliegende und außenliegende **34** Ausformungen besitzt, um die Reinigungsflüssigkeit seitlich sowie mittig zu verteilen. Die untere Öffnung öffnet sich durch einen trichterförmigen Kanal **32** nach oben. Die Abbildung **E** zeigt in Seitenansicht die Strömungshindernisse **19** mit einem trichterförmigen Kanal **32** und einer unteren Öffnung **31**, in der die Flüssigkeit aus dem Adapterkanal einströmt. Wie obig ausgeführt, kann der Kanal **32** unterschiedliche Ausformungen wie Kanäle oder ähnliches aufweisen oder mit einer Scheibe **29** mit Öffnungen kombiniert sein, um Flüssigkeit zu verteilen und umzulenken. Zur besseren Festlegung in einem Adapter oder einer Spülhülse besitzt der Trichter **19** einen seitlichen Randbereich, der auch Ausformungen aufweisen kann oder der alternativ auch eine Scheibe sein kann. Denkbar ist aber auch ein Hindernisteil, das zylindrisch ausgeführt ist und zusätzlich mehrere seitliche oder innenliegende trichterförmige Kanäle besitzt.

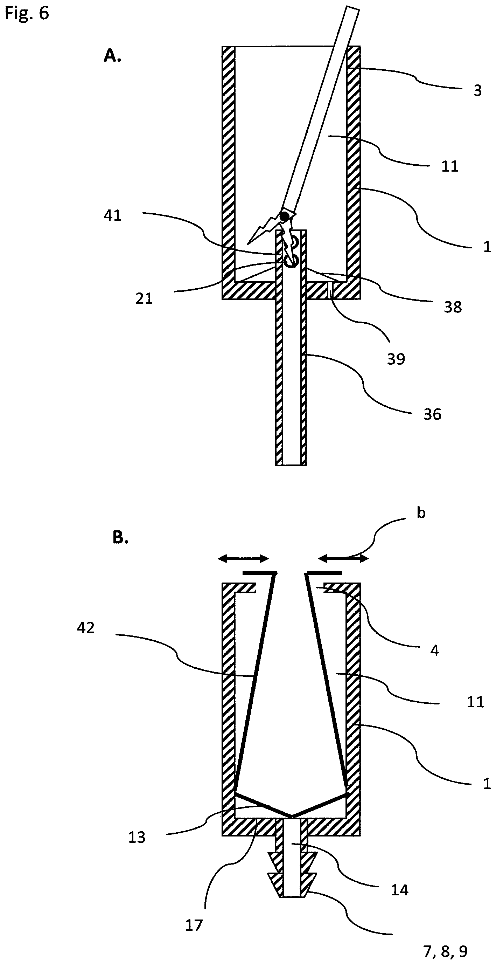

Fig. 6 zeigt in **A** und **B** jeweils eine Spülhülse mit einem Anschlussteil. Die dargestellte Spülhülse **A**. besitzt eine lange Zuleitung **36**, die sich über dem Hülsenboden erhebt sowie seitliche Öffnungen **21** und eine mittige Öffnung besitzt, um Reinigungsmedium auszuleiten. Alternativ kann die Hülse eine Kappe mit einer verengten Öffnung besitzen. Im Bodenbereich befindet sich eine Erhebung **38**, um Flüssigkeit nach außen zu lenken. Hier kann eine kleine Entlastungsöffnung **39** am Hülsenboden vorgesehen sein, die die Hülse **1** immer leer laufen lässt. Das herausstehende Zuleitungssteil, als ein Rohrstück **41** oder ähnliches mit Öffnungen **21**, ragt in den Hülsenraum **11**. Die seitlichen Öffnungen **21** können dabei ein Maulteil oder ein anderes Instrumententeil aufnehmen, offen halten und so effektiv reinigen. Damit vereinigt die Spülhülse wichtige Funktionen (wie z. B. eine Instrumentenhalterung, die Öffnung von Maulteilen, eine mittig Anordnung der zu reinigende Produkte und eine gute Medienverteilung), um insbesondere für sehr empfindliche und komplex gebaute Instrumententeile und Instrumente **3** effektiv reinigen zu können. In Abbildung **B**. ist eine Spülhülse gezeigt, deren Öffnung **4** sich nach oben hin verengt. Der Hülsenboden **17** ist flach ausgeführt. In die Hülse **1** ist ein innenliegendes Strömungshindernis in Ausgestaltung einer Haltefeder **42** oder eines Haltebleches eingebracht, das sich bei Einbringen eines Produktes weiten kann (gezeigt durch die Pfeile b). Das Strömungshindernis **42** läuft unten spitz **13** zu und überdeckt mittig den Anschlusskanal **14** des Anschlussadapters, welches ein Schlauchanschluss **9**, ein Steckteil **8** oder ein Gewinde **7** oder ähnliches sein kann. Von Vorteil ist, wenn die Hülse **1** elastisch ist, und die Rückstellkraft der Haltefeder **42** zusätzlich unterstützt. Damit kann das Strömungshindernis Produktschäfte effektiv halten und verhindern, dass besonders leichte Produktteile durch den Flüssigkeitsstrom herausgehoben werden.

## Patentansprüche

1. Reinigungsadapter zum Reinigen von Produkten, insbesondere für die Produktaufbereitung von medizinischen Instrumenten, der über eine Zuleitung oder über einen Flüssigkeitsverteiler mit Reinigungsmedium versorgt wird und einen vorderen Spülraum (11) aufweist, um hiermit zu reinigende Produkte (3) oder Produktteile aufzunehmen, **dadurch gekennzeichnet, dass** zumindest ein Strömungshindernis im Adapter in Form von wand- und/oder bodenständige Materialausformungen (18, 38, 44) und/oder in Form eines räumliches Strömungshindernis (12, 19, 26, 29, 42, 45) vorhanden ist, um den Reinigungsflüssigkeitsaustritt aus dem Adapterkanal (14) so zu ändern, dass diese für die Produktkanal- und die Produktaußenreinigung eines Hohlraumproduktes oder zumindest für die Produktaußenreinigung mehrerer Produkte nutzbar ist.

2. Reinigungsadapter nach Anspruch 1, **dadurch gekennzeichnet, dass** das Strömungshindernis so angeordnet ist, das die Flüssigkeitsverteilung im Adapter mittig und seitlich verteilt, gelenkt oder aufgeteilt wird und dabei ein Teil der Reinigungsflüssigkeit zumindest für die Produktkanalreinigung und ein anderer Teil für die Produktaußenflächenreinigung nutzbar ist und/oder dabei ein Verschluss des Adapteraustrittskanals (14) durch eines oder mehrere eingebrachten Produkte (3) verhindert wird.

3. Reinigungsadapter nach Anspruch 1, **dadurch gekennzeichnet, dass** dieser zumindest durch die innenliegenden Materialausformungen (18, 38, 44) oder Einsätze (12, 19, 26, 29, 42, 45) oder durch ein in den Adapterkanal (14) eingebrachtes Teil (13) eine Verwirbelung des Reinigungsmedium erzeugt wird.

4. Reinigungsadapter nach einem der Ansprüche 1 bis 3, **dadurch gekennzeichnet, dass** im Spülraum (11) die Außenflächen mehrerer Produktschäfte oder mehrerer Produkte (3) über zumindest einen innenliegenden Halteeinsatz und/oder über einen Hülsenabschluss (5) und/oder ein hausstehendes (42) Halteteil voneinander beabstandet sind und/oder gehalten werden, ohne dass dabei ein Produkt den Adapterkanal (14) verschließt und eine Reinigung der Produktaußenflächen erfolgt.

5. Reinigungsadapter nach einem der Ansprüche 1 bis 4, **dadurch gekennzeichnet, dass** als Strömungshindernisse Rillen (18) oder Materialerhöhungen und/oder Materialvertiefungen (34, 38, 44, 46) und/oder Einsätze (12, 19, 24, 26, 42, 45) mit Ausformungen (27, 28, 34, 13) oder Rohrteile (41) so eingesetzt sind und das keine vollständige Überdeckung eines Adapterkanals (14) durch ein zu reinigendes Produkt oder durch mehrere eingebrachten Produkte erfolgt.

6. Reinigungsadapter nach einem der voranstehenden Ansprüche, **dadurch gekennzeichnet, dass** der Spülraum (11) durch eine Hülse (1) gebildet wird und/oder dieser eine zusätzliche Kappe (5) mit einer verengten Öffnung (4) oder eine Haltefeder (42) aufweist und/oder der Spülraum (11) des Adapters innnlumige Erhebungen oder Rillen (22) aufweist, um Produkte mittig oder von einer Wandung beabstandet anzuordnen oder zu halten.

7. Reinigungsadapter nach einem der voranstehenden Ansprüche, **dadurch gekennzeichnet, dass** der Adapter eine obere Kappe (5) mit einer gezackten (22) oder unrunden (23) vorderen Öffnung (4) oder zumindest einen Steg (24) besitzt und/oder nach unten eine Klappe (25) oder ein Ventil (26) überdeckt.

8. Reinigungsadapter nach einem der voranstehenden Ansprüche, **dadurch gekennzeichnet, dass** dieser ein innenliegendes Strömungshindernis (12, 19, 29, 26, 42), ein Gitter (45), eine Spirale (12) oder eine Schnecke, eine Haltefeder (42), ein Filter, ein Trichter (19) eine Welle, ein Ventil (26) und/oder eine Scheibe (29) ist und dieses vom Adapterboden beabstandet oder zumindest seitlich von einer Adapterwandung gehalten wird.

9. Reinigungsadapter nach einem der voranstehenden Ansprüche, **dadurch gekennzeichnet, dass** dieser zumindest zweiteilig (1, 5, 6) ist und zum Anschluss für Reinigungslösungsleitungen einen Luer Lock Anschluss, ein Gewinde- (7), ein Bajonett-, ein Steck-(8) oder ein Schlauchteil (9) und nach vorne eine austauschbare Hülse (1) und/oder eine Kappe (5) besitzt und dieser modulare Aufbau in Verbindung mit Flüssigkeitsverteilern wie Spülleisten oder Spülzylindern zum reinigen von medizinischen Hohlraumprodukten (3) in Reinigungsmaschinen und Ultraschallbädern einsetzbar ist.

10. Reinigungsadapter nach einem der voranstehenden Ansprüche, **dadurch gekennzeichnet, dass** zumindest vor oder im Austrittskanal (14) des Adapters oder einer Hülse (1), ein Drahtteil (13), ein Federteil (42), oder ein Formteil in Form eines kugliges oder kegliges Endstück oder ein drehbares Teil in Form einer Kugel, einer Welle oder einer Turbine mittig angeordnet ist, um eine turbulente Strömung zu erzeugen.

11. Reinigungsadapter nach einem der voranstehenden Ansprüche, **dadurch gekennzeichnet, dass** die eingebrachten Hohlraumprodukte (3) und dessen Kanäle durch die trichterförmigen inneren Strukturen (12, 19, 26, 29) im Adapter auch im Liegen oder schräggestellt sicher gereinigt und/oder zusätzlich durch eine Kappe (5) mittig im Spülraum (11) platziert werden.

12. Reinigungsadapter nach einem der voranstehenden Ansprüche, **dadurch gekennzeichnet, dass** dieser in der Hülse (1) eine Verengung durch eine Manschette (2) oder durch Ausformungen in der Hülse zur besseren Halterung der zu reinigenden Produkte (3) und/oder zur Erzeugung eines Venturi-Effekts besitzt, um die Reinigungsleistung zu verbessern.

13. Spülhülse (1) in Ausgestaltung eines Reinigungsadapters zum Reinigen von Produkten insbesondere für die Produktaufbereitung von medizinischen Instrumenten, die ein Anschlussteil (7, 8, 9) oder eine Zuleitung (36) für Reinigungsflüssigkeit besitzt und zu reinigende Produkte oder Produktteile (3) aufnimmt, **dadurch gekennzeichnet, dass** die Spülhülse Erhebungen am Spülhülsenboden (17, 46) oder überströmbare innenliegende Teile (12, 19, 26, 29,45) oder ein herausstehendes Rohrteil (41) mit seitlichen Öffnungen (21) oder ein Halteteil (5, 42) besitzt, um Reinigungsflüssigkeit zu verteilen, um damit die Produktkanal- und Produktaußenreinigung zu verbessern.

14. Spülhülse nach Anspruch 13, **dadurch gekennzeichnet, dass** diese ein einziges Formteil ist und/oder zumindest ein herausnehmbares innenliegendes Teil (12, 19, 29, 42) enthält, das ein Flüssigkeitsfilter, ein Gitter (45), eine Haltefeder (42), ein Ventil (26) oder eine Klappe (25) ist oder Ausformungen (18, 38, 46) oder Strukturen (13, 22, 23, 24) besitzt.

15. Spülhülse nach einem der Ansprüche 13 bis 15, **dadurch gekennzeichnet, dass** eine elastische Hülse (1) eingesetzt ist um eine Feder (12, 42) zu halten oder anzudrücken und um damit die Haltekraft und/oder die Rückstellkraft der Feder zusätzlich zu erhöhen oder zu verbessern zumindest wenn ein Produkt (3) eingebracht ist.
